# EUROPEAN PATENT APPLICATION

(11) **EP 3 785 694 A1**
(43) Date of publication of application: **03.03.2021**
(21) Application number: 19194750.6
(22) Date of filing: 30.08.2019
(51) Int. Cl.: A61K 8/04, A61K 8/34, A61K 8/49, A61K 8/73, A61K 8/81, A61K 8/891, A61Q 1/10

(54) **EYELINER PRODUCT AND METHOD THEREOF**

(71) Applicant: Hangzhou Ocean Pearl Industrial Co., Ltd., 310052 Hangzhou, Guangdong (CN)
(72) Inventor: HUANG, Juanxiu, HANGZHOU, Zhejiang 310000 (CN)
(74) Representative: Novagraaf Technologies

(57) **Abstract**

The present disclosure provides an eyeliner product and a method thereof. The eyeliner product includes components as follows: foundation makeup liquid, acrylic acid octyl acrylamide copolymer or propylene ester octyl acrylamide copolymer, tackifier, vegetable gum, and surface modifier. The eyeliner product is provided by weight. The acrylic acid octyl acrylamide copolymer or the propylene ester octyl acrylamide copolymer is provided in an amount ranging from 10-11 parts. The tackifier is provided in an amount ranging from 1-3 parts. The vegetable gum is provided in an amount ranging from 6-12 parts. The surface modifier is provided in an amount ranging from 0.1-2 parts. And the vegetable gum is starch glue.

## Description

### TECHNICAL FIELD

The present disclosure relates to a field of cosmetic technology, and in particular to an eyeliner product with a bonding function.

### BACKGROUND

At present, most eyeliner products on the market are pen-like beauty tools with black or colored liquids. When using the eyeliner products, eyeliner is applied to upper eyelids near roots of eyelashes to modify and beautify eyes.

Conventional eyeliner products mostly consist of organic components, and their roles are only for personalized modification and beauty, and have no additional functions. However, such eyeliner products are unable to fix false eyelashes. With enrichment of beauty products and upgrading of technology, there is a need for an eyeliner product that not only takes beauty of the eyes into account, but also comes with special functions, such as auxiliary fixing the false eyelashes. Conventional eyeliner products and other similar beauty products are unable to realize this function. For this reason, the present disclosure provides an eyeliner product with a bonding function.

### SUMMARY

The present disclosure provides an eyeliner product with bonding function to solve a technical problem set forth in the above prior art.

To achieve the above object, the present disclosure provides an eyeliner product. The eyeliner product include components as follows: foundation makeup liquid, acrylic acid octyl acrylamide copolymer or propylene ester octyl acrylamide copolymer, tackifier, vegetable gum, and surface modifier.

The eyeliner product is provided in parts by weight. The acrylic acid octyl acrylamide copolymer or the propylene ester octyl acrylamide copolymer is provided in an amount ranging from 10-11 parts. The tackifier is provided in an amount ranging from 1-3 parts. The vegetable gum is provided in an amount ranging from 6-12 parts. The surface modifier is provided in an amount ranging from 0.1-2 parts. And the vegetable gum is starch glue.

Furthermore, the foundation makeup liquid includes following components in parts by weight:,
deionized water provided in an amount ranging from 44-45 parts,
propylene glycol provided in an amount ranging from 5-6 parts,
ethylhexylglycerin provided in an amount ranging from 0.1-0.5 parts,
butanediol provided in an amount ranging from 3-4 parts,
PEG-3 sorbitan oleate provided in an amount ranging from 1-2 parts,
stearyl ether-21 provided in an amount ranging from1-2 parts,
polydimethylsiloxane provided in an amount ranging from 8-9 parts,
octyl dodecanol provided in an amount ranging from 10-11 parts,
polyvinyl pyrrolidone provided in an amount ranging from 2-3 parts,
sorbitan sesquioleate provided in an amount of 1-2 parts,
chlorphenesin provided in an amount ranging from 0.1-0.5 parts, and
essence provided in an amount ranging from 0.01-0.05 parts.

Furthermore, the foundation makeup liquid further comprises preservative provided in an mount ranging from 0.1-1 parts by weight.

Furthermore, the preservative is selected from at least one of phenoxyethanol, nipagin ester, methylparaben, and ethylparaben.

The present disclosure further provides a method for preparing an eyeliner product. The method includes steps:
A: heating, mixing, and uniformly stirring vegetable gum with surface modifier, tackifier, and acrylic acid octyl acrylamide copolymer or propylene ester octyl acrylamide copolymer to form a viscous raw material;
B: mixing foundation makeup liquid including deionized water, propylene glycol, ethylhexylglycerin, butanediol, PEG-3 sorbitan oleate, stearyl ether-21, polydimethylsiloxane, octyl dodecanol, polyvinyl pyrrolidone, sorbitan sesquioleate , and chlorphenesin with oil-water phase raw material and the viscous raw material to form a mixture; and
C: stirring and heating the mixture to 90°C until the mixture is completely dissolved and evenly dispersed, then uniformly stirring and emulsifying the mixture at 90°C for 30 minutes, adding pigment into the mixture and preforming a vacuum defoamation on the mixture after the mixture is cooled to 70°C, and adding essence and preservative into the mixture after the mixture is cooled to 50°C.

Furthermore, the pigment is previously ground and powdered.

In the present disclosure, adhesiveness of the eyeliner product is realized by the viscous raw material. The eyeliner product includes the natural vegetable gum, which reduces an irritation of the eyeliner product. At the same time, the surface modifier is able to reduce a hydrophilicity of the vegetable gum, increase a water resistance of the eyeliner product, and improve the hydrophilicity of the vegetable gum. The eyeliner product is very sticky for a period of time after applying the eyeliner product to the eyeliner position of the face. However, as time passes, viscosity of the eyeliner product gradually decreases, such that the eyeliner product is configured to attach and fix false eyelashes. The eyeliner product of the present disclosure has a new additional function, and has an ability to fix any types of false eyelashes, and is convenient to use, and further, is able to replace existing false eyelash fixing methods. The eyeliner product is not limited to eyeliner pen, eyeliner liquid, eyeliner grease, eyeliner powder, eyeliner glue, eyeliner glue pen, eyeliner stickers, etc.. Similarly, the eyeliner product is able to be applied to any of the above eyeliner products. Further, the eyeliner product is able to fix other small makeup items except for the false eyelashes.

### BRIEF DESCRIPTION OF DRAWINGS

The drawings are included to provide a further understanding of embodiments of the present disclosure, which form portions of the specification and are used to illustrate the present disclosure along with embodiments of the present disclosure, and is not intended to limit the present disclosure.

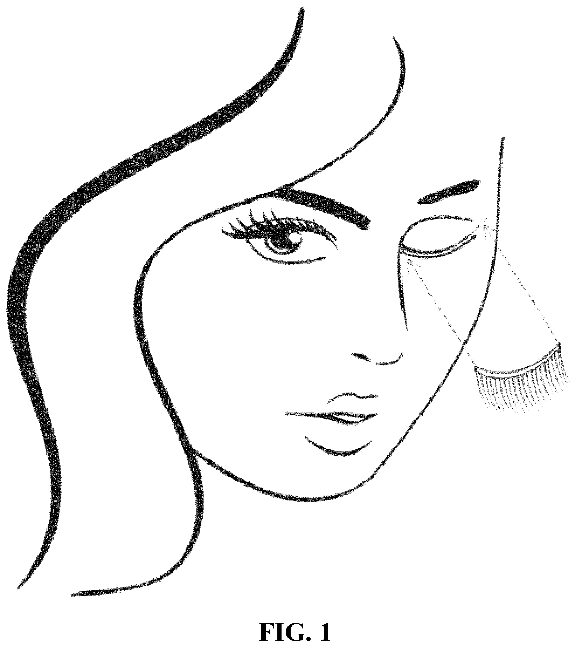

FIG. 1 is a schematic diagram showing a fixing of false eyelashes by the eyeliner product of the present disclosure.

### DETAILED DESCRIPTION

The technical solutions in the embodiments of the present disclosure are clearly and completely described in the following with reference to the accompanying drawings in the embodiments of the present disclosure. It is obvious that the described embodiments are only a part of the embodiments of the present disclosure, but not all embodiments. All other embodiments obtained by those skilled in the art based on the embodiments of the present disclosure without creative efforts are within the scope of the present disclosure.

It should be understood in the description of the present disclosure that terms such as "central", "horizontal", "upper", "lower", "left", "right", "vertical", "horizontal", "top", "bottom", "inner", "outer", etc. indicate direction or position relationships shown based on the drawings, and are only intended to facilitate the description of the present disclosure and the simplification of the description rather than to indicate or imply that the indicated device or element must have a specific direction or constructed and operated in a specific direction, and therefore, shall not be understood as a limitation to the present disclosure.

It should be noted in the description of the present disclosure that, unless otherwise regulated and defined, terms such as "installation," "bonded" , "connect", and "bonding" shall be understood in broad sense, and for example, may refer to fixed bonding or detachable bonding or integral bonding; may refer to mechanical bonding or electrical bonding; and may refer to direct bonding or indirect bonding through an intermediate medium or inner communication of two elements. For those of ordinary skill in the art, the meanings of the above terms in the present disclosure may be understood according to concrete conditions.

As shown in FIG. 1, the present disclosure provides an eyeliner product. The eyeliner product includes components in parts by weight
acrylic acid octyl acrylamide copolymer or propylene ester octyl acrylamide copolymer provided in an amount ranging from 10-11 parts,
tackifier provided in an amount ranging from 1-3 parts,
vegetable gum provided in an amount ranging from 6-12 parts, and
surface modifier provided in an amount ranging from 0.1-2 parts.

And the vegetable gum is starch glue.

The foundation makeup liquid includes following components in parts by weight:
deionized water provided in an amount ranging from 44-45 parts,
propylene glycol provided in an amount ranging from 5-6 parts,
ethylhexylglycerin provided in an amount ranging from 0.1-0.5 parts,
butanediol provided in an amount ranging from 3-4 parts,
PEG-3 sorbitan oleate provided in an amount ranging from 1-2 parts,
stearyl ether-21 provided in an amount ranging from1-2 parts,
polydimethylsiloxane provided in an amount ranging from 8-9 parts,
octyl dodecanol provided in an amount ranging from 10-11 parts,
polyvinyl pyrrolidone provided in an amount ranging from 2-3 parts,
sorbitan sesquioleate provided in an amount of 1-2 parts,
chlorphenesin provided in an amount ranging from 0.1-0.5 parts, and
essence provided in an amount ranging from 0.01-0.05 parts.

### Embodiment 1

The present disclosure provides an eyeliner product with a bonding function. In the embodiment, the eyeliner product is an eyeliner liquid, components of the eyeliner liquid are provided by weight. A method for preparing the eyeliner liquid includes steps as follows,
A: heating 10 parts of vegetable gum with 1.8 parts of surface modifier, 2.4 parts of tackifier, and 11 parts of acrylic acid octyl acrylamide copolymer or propylene ester octyl acrylamide copolymer to 60° C, mixing and uniformly stirring them, such that the vegetable gum is modified to reduce hydrophilicity, and viscosity is increased by the tackifier, and the vegetable gum is mixed with the acrylic acid octyl acrylamide copolymer or the propylene ester octyl acrylamide copolymer mixing to form a viscous raw material;
B: mixing 44.54 parts of deionized water, 5.2 parts of propylene glycol, 0.2 parts of ethylhexylglycerin, 3.6 parts of butanediol, 1.2 parts of PEG-3 sorbitan oleate, 1.8 parts of stearyl ether-21, 8.6 parts of polydimethylsiloxane, 10.6 parts of octyl dodecanol, 2.1 parts of polyvinyl pyrrolidone, 1.6 parts of sorbitan sesquioleate, and 0.2 parts of chlorphenesin with oil-water phase raw material and the viscous raw material to form a mixture; and
C: stirring and heating the mixture to 90°C until the mixture is completely dissolved and evenly dispersed, then uniformly stirring and emulsifying the mixture at 90°C for 30 minutes, adding pigment that is previously ground into the mixture and preforming a vacuum defoamation on the mixture after the mixture is cooled to 70°C, and adding 0.05 parts of essence and 0.5 parts of preservative into the mixture after the mixture is cooled to 50°C, where the preservative is phenoxyethanol; cooling the mixture to 35°C to form the eyeliner liquid; and filling the eyeliner liquid into a eyeliner pen.

### Embodiment 2

The present disclosure provides an eyeliner product with bonding function. In the embodiment, the eyeliner product is eyeliner liquid, where components of the eyeliner liquid are provided by weight. A method for preparing the eyeliner liquid includes steps as follows,
A: heating 6 parts of vegetable gum with 0.2 parts of surface modifier, 1 parts of tackifier, and 10 parts of acrylic acid octyl acrylamide copolymer or propylene ester octyl acrylamide copolymer to 60° C, mixing and uniformly stirring them, such that the vegetable gum is modified to reduce hydrophilicity, and viscosity is increased by the tackifier, and the vegetable gum is mixed with the acrylic acid octyl acrylamide copolymer or the propylene ester octyl acrylamide copolymer mixing to form a viscous raw material;
B: mixing 44.54 parts of deionized water, 5.2 parts of propylene glycol, 0.2 parts of ethylhexylglycerin, 3.6 parts of butanediol, 1.2 parts of PEG-3 sorbitan oleate, 1.8 parts of stearyl ether-21, 8.6 parts of polydimethylsiloxane, 10.6 parts of octyl dodecanol, 2.1 parts of polyvinyl pyrrolidone, 1.6 parts of sorbitan sesquioleate, and 0.2 parts of chlorphenesin with oil-water phase raw material and the viscous raw material to form a mixture; and
C: stirring and heating the mixture to 90°C until the mixture is completely dissolved and evenly dispersed, then uniformly stirring and emulsifying the mixture at 90°C for 30 minutes, adding pigment that is previously ground into the mixture and preforming a vacuum defoamation on the mixture after the mixture is cooled to 70°C, and adding 0.05 parts of essence and 0.5 parts of preservative into the mixture after the mixture is cooled to 50 °C, where the preservative are phenoxyethanol; cooling the mixture to 35°C to form the eyeliner liquid; and filling the eyeliner liquid into a eyeline pen.

The material and processing steps of embodiment 2 are same as the material and processing steps of the embodiment 1, except that the ratio of the components is different.

### Embodiment 3

The present disclosure provides an eyeliner product with bonding function. In the embodiment, the eyeliner product is an eyeliner liquid, where components of the eyeliner liquid are provided by weight. A method for preparing the eyeliner liquid includes steps as follows,
A: heating 8 parts of vegetable gum with 1.4 parts of surface modifier, 1.8 parts of tackifier, and 10.6 parts of acrylic acid octyl acrylamide copolymer or propylene ester octyl acrylamide copolymer to 60° C, mixing and uniformly stirring them, such that the vegetable gum is modified to reduce hydrophilicity, and viscosity is increased by the tackifier, and the vegetable gum is mixed with the acrylic acid octyl acrylamide copolymer or the propylene ester octyl acrylamide copolymer mixing to form a viscous raw material;
B: mixing 44.54 parts of deionized water, 5.2 parts of propylene glycol, 0.2 parts of ethylhexylglycerin, 3.6 parts of butanediol, 1.2 parts of PEG-3 sorbitan oleate, 1.8 parts of stearyl ether-21, 8.6 parts of polydimethylsiloxane, 10.6 parts of octyl dodecanol, 2.1 parts of polyvinyl pyrrolidone, 1.6 parts of sorbitan sesquioleate, and 0.2 parts of chlorphenesin with oil-water phase raw material and the viscous raw material to form a mixture; and
C: stirring and heating the mixture to 90°C until the mixture is completely dissolved and evenly dispersed, then uniformly stirring and emulsifying the mixture at 90°C for 30 minutes, adding pigment that is previously ground into the mixture and preforming a vacuum defoamation on the mixture after the mixture is cooled to 70°C, and adding 0.05 parts of essence and 0.5 parts of preservative into the mixture after the mixture is cooled to 50 °C, where the preservative is phenoxyethanol; cooling the mixture to 35°C to form the eyeliner liquid; and filling the eyeliner liquid into a eyeline pen.

The material and processing steps of embodiment 3 are same as the material and processing steps of the embodiments 1 and 2, except that the ratio of the components is different.

The eyeliner products prepared according to the ratios of the components and preparation methods described in the embodiments 1-3 are subjected to the wearing test with the false eyelashes, and results are evaluated by A, B, and C, where A is a correspondingly strong or large value, C is a corresponding weak and small value relative to A, as shown in the following table:

| | Embodiment 1 | Embodiment 2 | Embodiment 3 |
|---|---|---|---|
| Viscosity | A | C | B |
| Time spent on drying the eyeliner product | A | C | B |

The viscosity of the eyeliner product of embodiment 1 is the strongest, and the time for drying the eyeliner product of embodiment 1 is the longest. The viscosity of the eyeliner product of embodiment 2 is the worst, and the time for drying the eyeliner product of embodiment 2 is the shortest. The viscosity of the eyeliner product of embodiment 3 is the most moderate, and the time for drying the eyeliner product of embodiment 3 is the most moderate.

At a time of makeup, an eyeliner portion of the face is evenly painted by the eyeliner pen having the eyeliner product. And the eyeliner product is viscous for a period of time after the painting is finished, so that the false eyelashes are fixed to the eyeliner portion of the face by the eyeliner product. The time for drying the eyeliner product in the embodiment 2 is between the time spent on drying the eyeliner product in embodiment 1 and the time spent on drying the eyeliner product in embodiment 3. If the time for drying the eyeliner product exceeds the period of time, the viscosity of the eyeliner product is reduced, or even the eyeliner product is no longer viscous, that is, a standard for fixing the false eyelashes cannot be reached.

In the embodiment 3, the amount of the acrylic acid octyl acrylamide copolymer or the propylene ester octyl acrylamide copolymer, the tackifier, the vegetable gum, and the surface modifier is appropriate and the eyeliner product prepared by the formula has moderate viscosity, and the eyeliner portion is mot uncomfortable during applying, and the false eyelashes are fixed well and would not be loose.

At the same time, if it is not necessary to put on the false eyelashes, after waiting for a long period of time and the eyeliner product is dry, the viscosity of the eyeliner area is reduced, or even the eyeliner area is no longer sticky, so that the eyeliner product is able to be used as an ordinary eyeliner product to beautify the eyeliner.

The eyeliner product is not limited to eyeliner pen, eyeliner liquid, eyeliner grease, eyeliner powder, eyeliner glue, eyeliner glue pen, eyeliner stickers, etc.. The eyeliner product in these embodiments of the present disclosure is suitable for widespread use, and viscous technology of formulations of the eyeliner product is able to be transplanted to other beauty products. For example, the eyeliner product is able to fix other small makeup items except for the false eyelashes.

While the embodiments of the present disclosure have been shown and described, it will be apparent to those skilled in the art that a number of simple deductions or substitutions may be made without departing from the principle and conception of the present disclosure, which should be considered as being within the scope of the present disclosure.

## Claims

1. An eyeliner product, comprising components as follows:
foundation makeup liquid;
acrylic acid octyl acrylamide copolymer or propylene ester octyl acrylamide copolymer;
tackifier;
vegetable gum; and
surface modifier;
wherein the eyeliner product is provided in parts by weight; the acrylic acid octyl acrylamide copolymer or the propylene ester octyl acrylamide copolymer is provided in an amount ranging from 10-11 parts, the tackifier is provided in an amount ranging from 1-3 parts; the vegetable gum is provided in an amount ranging from 6-12 parts; and the surface modifier is provided in an amount ranging from 0.1-2 parts; and the vegetable gum is starch glue.

2. The eyeline product according to claim 1, wherein the foundation makeup liquid comprises following components in parts by weight:
deionized water provided in an amount ranging from 44-45 parts,
propylene glycol provided in an amount ranging from 5-6 parts,
ethylhexylglycerin provided in an amount ranging from 0.1-0.5 parts,
butanediol provided in an amount ranging from 3-4 parts,
PEG-3 sorbitan oleate provided in an amount ranging from 1-2 parts,
stearyl ether-21 provided in an amount ranging from1-2 parts,
polydimethylsiloxane provided in an amount ranging from 8-9 parts,
octyl dodecanol provided in an amount ranging from10-11 parts,
polyvinyl pyrrolidone provided in an amount ranging from 2-3 parts,
sorbitan sesquioleate provided in an amount ranging from 1-2 parts,
chlorphenesin provided in an amount ranging from 0.1-0.5 parts, and
essence provided in an amount ranging from 0.01-0.05 parts.

3. The eyeline product according to claim 1, wherein the foundation makeup liquid further comprises preservatives provided in an amount ranging from 0.1-1 parts by weight.

4. The eyeline product according to claim 3, wherein the preservatives are selected from at least one of phenoxyethanol, nipagin ester, methylparaben, and ethylparaben.

5. A method for preparing an eyeliner product, comprising steps:
heating, mixing, and uniformly stirring vegetable gum with a surface modifier, tackifier, and acrylic acid octyl acrylamide copolymer or propylene ester octyl acrylamide copolymer to form a viscous raw material;
mixing foundation makeup liquid including deionized water, propylene glycol, ethylhexylglycerin, butanediol, PEG-3 sorbitan oleate, stearyl ether-21, polydimethylsiloxane, octyl dodecanol, polyvinyl pyrrolidone, sorbitan sesquioleate , and chlorphenesin with oil-water phase raw material and the viscous raw material to form a mixture; and
stirring and heating the mixture to 90°C until the mixture is completely dissolved and evenly dispersed, then uniformly stirring and emulsifying the mixture at 90°C for 30 minutes, adding pigment into the mixture and preforming a vacuum defoamation on the mixture after the mixture is cooled to 70°C, and adding essence and preservatives into the mixture after the mixture is cooled to 50°C.

6. The method according to claim 5, wherein the pigment is previously ground and powdered.
